# EUROPÄISCHE PATENTSCHRIFT

(11) **EP 0 401 626 B1**
(45) Veröffentlichungstag und Bekanntmachung des Hinweises auf die Patenterteilung: **19.01.1994**
(21) Anmeldenummer: 90110029.7
(22) Anmeldetag: 26.05.1990
(51) Int. Cl.: C07C 43/29, C07C 41/16

(54) **Verfahren zur Herstellung chlorierter Diphenylether**
Process for the preparation of chlorinated diphenyl ether
Procédé pour la préparation d'éther diphényl chloré

(30) Priorität: 09.06.1989 DE 3918897
(43) Veröffentlichungstag der Anmeldung: 12.12.1990
(73) Patentinhaber: BAYER AG, 51368 Leverkusen (DE)
(72) Erfinder: Klein, Alfons, D-4000 Düsseldorf (DE); Fiege, Helmut, Dr., D-5090 Leverkusen 1 (DE); George, Jochim, Dr., D-5090 Leverkusen 3 (DE)

(56) Entgegenhaltungen:
- EP-A- 0 051 235
- EP-A- 0 297 028
- FR-A- 1 576 298

## Beschreibung

Die Erfindung betrifft ein Verfahren zur Herstellung chlorierter Diphenylether durch Umsetzung der zugrunde liegenden Alkaliphenolate mit Dichlorbenzolen in Abwesenheit von Metallkatalysatoren.

Diese Umsetzung ist ohne Katalysator bisher lediglich für den Fall bekannt geworden, daß sowohl das Alkaliphenolat mit Kalium als dem Kation als auch das Chlorbenzol als Beispiel für ein Halogenbenzol durch je eine Nitrogruppe stark aktiviert waren, wobei mindestens eine der Nitrogruppen o- oder p-ständig zur Phenolat- bzw. Chlorgruppe stehen muß. Die Reaktion wurde in siedendem Dimethylformamid (DMF) durchgeführt und ergab für die verschiedenen Stellungsisomeren Ausbeuten von 60 bis 72 % der theoretischen Ausbeute (J. Org. Chem. 27 (1962), 4098).

Die Ausdehnung auf nicht aktivierte Phenolate und Halogenbenzole ist als sogenannte Ullmann-Reaktion bekannt (Krauch-Kunz "Namenreaktionen in der organischen Chemie", D. Hütig Verlag GmbH, Heidelberg, 2. Auflage 1962, S. 474). Diese Reaktion besteht in der Umsetzung von Alkaliphenolaten mit Arylhalogeniden in Gegenwart von Kupfer oder Kupferverbindungen als Katalysator. Diese Ullmann-Reaktion ist ihrerseits widerum in einer Reihe verschiedener Ausführungsformen bekannt.

So ist bekannt, 1,4-Dichlorbenzol mit 3-Chlorphenol unter CuCl/KJ-Katalyse zu 3,4'-Dichlordiphenylether umzusetzen (US 3.472.782, US 3.371.120).

Eine Verbesserung des Ullmann-Katalysators wird in EP 51 235 durch Verwendung basischer Kupfercarbonate oder Kupfersalze niederer aliphatischer Carbonsäuren vorgeschlagen.

Weiterhin ist bekannt, chlorierte Diphenylether aus Chlorphenolaten und Dichlorbenzolen in Gegenwart eines Kupferkatalysators und eines aprotischen Lösungsmittels als Co-Katalysator bei Temperaturen von 120 bis 220°C herzustellen (EP 297 028).

Aus FR-A 1 576 298 ist die Herstellung von Arylethern bekannt, die in einem Arylteil zwei Halogenatome enthalten, durch Umsetzung eines Trihalogenbenzols mit einem Alkaliphenolat in Gegenwart eines inerten, stark polaren Lösungsmittels bei erhöhter Temperatur.

Aus EP-A 297 028 ist bekannt, Fluordiphenylether herzustellen, die in einem Arylteil (oder beiden) ein Chloratom enthalten, durch Umsetzung eines Dichlorbenzols einem Alkaliphenolat in Gegenwart eines Kupferkatalysators und eines aprotischen Lösungsmittels bei 120 bis 220°C. Da ein Chloratom in einem Trichlorbenzol reaktiver ist als Chloratom in einem Dichlorbenzol, liegt der Stand der Technik also nahe, beim Einsatz von Trichlorbenzolen keine Katalysatoren einzusetzen, wohl aber beim Einsatz von Dichlorbenzolen Katalysatoren zu verwenden.

Es wurde ein Verfahren zur Herstellung von chlorierten Diphenylethern der Formel 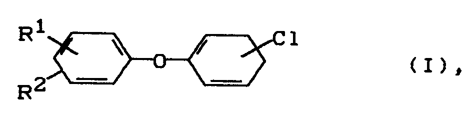
worin
- R¹: für Wasserstoff, C₁-C₆-Alkyl, C₃-C₇-Cycloalkyl oder C₁-C₆-Alkoxy,
- R²: für Wasserstoff oder Chlor stehen,
gefunden, das dadurch gekennzeichnet ist, daß man 1 Mol eines Phenolats der Formel 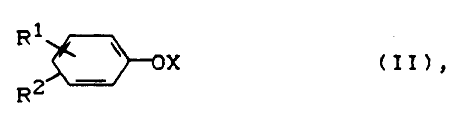
worin
- X: ein Alkalimetallkation ist und
- R¹ und R²: die obige Bedeutung haben,
mit 1,1 bis 10 Mol eines Dichlorbenzols der Formel 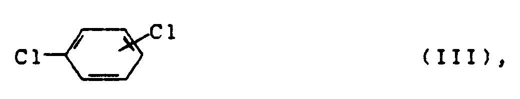
in einem polaren aprotischen Lösungsmittel im Temperaturbereich von 190 bis 240°C, bevorzugt 205 bis 225°C, in Abwesenheit von Metallkatalysatoren umsetzt.

Es war überraschend und nicht vorauszusehen, daß bei der erfindungsgemäßen Verwendung eines polaren aprotischen Lösungsmittels in Abwesenheit von Metallkatalysatoren, insbesondere in Abwesenheit eines Kupferkatalysators, Phenolate mit nicht aktivierten Halogenbenzolen zu den entsprechenden Diphenylethern mit ausgezeichneter Ausbeute umgesetzt werden können.

Die Vorteile des neuen Verfahrens sind seine relativ einfache Durchführbarkeit und die durch die sehr gute Ausbeute bedingte hohe Wirtschaftlichkeit.

Bei der Durchführung der Diphenylether-Herstellung nach Methoden, die dem Stand der Technik entsprechen, sind aufwendige Maßnahmen zur Entsorgung der zwangsweise anfallenden kupferhaltigen Abwässer erforderlich. Es ist ein besonderer Vorteil des erfindungsgemäßen Verfahrens, daß, bedingt durch seinen kupferfreien Ablauf, solche zusätzlichen Arbeiten und Aufwendungen entfallen.

Polare aprotische Lösungsmittel für das erfindungsgemäße Verfahren sind unter anderem: Dimethylformamid (DMF), Dimethylacetamid, Diethylformamid, Diethylacetamid, Hexamethylphosphorsäuretriamid, N-Methyl-pyrrolidon (NMP), Dimethylsulfoxid (DMSO), Sulfolan, Diethylenglykol-dimethylether, Glykol-dimethylether, Ethylencarbonat, Propylencarbonat und andere, in bevorzugter Weise DMF, Dimethylacetamid, Hexamethylphosphorsäuretriamid, NMP, DMSO, Sulfolan, Dimethylenglykol-dimethylether, Glykol-dimethylether, Ethylencarbonat und Propylencarbonat, besonders bevorzugt NMP, DMF, Dimethylacetamid und DMSO. Solche Lösungsmittel können sowohl einzeln als auch im Gemisch eingesetzt werden.

Die Menge des erfindungsgemäß einzusetzenden polaren aprotischen Lösungsmittels kann in weiten Bereichen schwanken. Sie richtet sich vor allem nach der Löslichkeit des Phenolats (II). Im allgemeinen liegt die Menge des Lösungsmittels bei 1,5 bis 10 Gew.-Teilen, bevorzugt bei 2 bis 6 Gew.-Teilen pro Gew.-Teil des eingesetzten Phenolats (II).

Die erfindungsgemäß einzusetzenden Phenolate der Formel (II) sind bevorzugt die Natrium- oder Kaliumsalze, besonders bevorzugt die Kaliumsalze, der zugrunde liegenden Phenole. In diesen bedeutet C₁-C₆-Alkyl Methyl, Ethyl, Propyl, Isopropyl, Butyl, Isobutyl, tert.-Butyl, Amyl, verzweigtes Amyl, Hexyl, verzweigtes Hexyl, bevorzugt die genannten C₁-C₄-Alkylreste, besonders bevorzugt Methyl oder Ethyl. C₃-C₇-Cycloalkyl ist hierin Cyclopropyl, Cyclobutyl, Cyclopentyl, Cyclohexyl, Cycloheptyl, Methyl-cyclopropyl, Dimethyl-cyclopropyl, Methyl-cyclopentyl, Dimethyl-cyclopentyl, Methyl-cyclohexyl, bevorzugt Cyclopropyl, Cyclopentyl oder Cyclohexyl oder deren Methyl-substituierten Abkömmlinge. C₁-C₆-Alkoxy bedeutet Methoxy, Ethoxy, Propoxy, Isopropoxy, Butoxy, Isobutoxy, tert.-Butoxy, Amyloxy, verzweigtes Amyloxy, Hexyloxy oder verzweigtes Hexyloxy, bevorzugt die genannten C₁-C₄-Alkoxyreste, besonders bevorzugt Methoxy oder Ethoxy.

Solche zugrunde liegenden Phenole sind unter anderem: Phenol, o-, m-, p-Kresol, o-, m-, p-Ethylphenol, o-, m-, p-Isopropylphenol, o-, m-, p-, tert.-Butylphenyl, o-, m-, p-Cyclohexylphenol, o-, m-, p-Chlorphenol, die verschiedenen isomeren Chlorkresole und andere.

Dichlorbenzole der Formel (III) sind: o-Dichlorbenzol, m-Dichlorbenzol, p-Dichlorbenzol.

Im erfindungsgemäßen Verfahren wird das Dichlorbenzol (III) im Überschuß zum Phenolat (II) verwendet. Das molare Verhältnis von Dihalogenbenzol zu Phenolat beträgt 1,1 - 10:1, bevorzugt 1,2 - 5:1, besonders bevorzugt 1,8 - 3,5:1.

Die Reaktionstemperatur des erfindungsgemäßen Verfahrens liegt im Bereich von 190 bis 240°C, vorzugsweise im Bereich von 205 bis 225°C. Die Umsetzung wird im allgemeinen bei Normaldruck durchgeführt. Selbstverständlich ist es auch möglich, insbesondere wenn der Siedepunkt des gewählten Lösungsmittels oder des Dichlorbenzols unterhalb der gewählten Reaktionstemperatur liegt, die Reaktion unter Druck durchzuführen. In einem solchen Fall wird bevorzugt unter dem automatisch sich einstellenden Eigendruck des Reaktionssystems gearbeitet.

Es wurde gefunden, daß es vorteilhaft ist, das erfindungsgemäße Verfahren in Gegenwart von zusätzlichem freien Phenol durchzuführen, wobei das freie Phenol das gleiche ist, welches dem Phenolat (II) zugrunde liegt.

Die Menge eines solchen anwesenden freien Phenols liegt im Bereich von 0,05 bis 0,6 Mol, bevorzugt 0,15 bis 0,4 Mol, freies Phenol pro Mol des Phenolats.

Zur Durchführung des erfindungsgemäßen Verfahrens kann auf übliche Techniken zurückgegriffen werden. Beispielsweise kann man die Reaktanten, möglichst unter wasserfreien Bedingungen, vorlegen und das Reaktionsgemisch mehrere Stunden, vielfach zwischen 8 und 20 Stunden, in jedem Falle aber abhängig auch von der Ansatzgröße, bei der gewünschten Temperatur rühren. In diesem Fall muß das Phenolat in einem getrennten Verfahrensschritt, beispielsweise aus Phenol und Alkalihydroxid, vorher hergestellt werden.

In einer vorteilhaften Variante des erfindungsgemäßen Verfahrens kann jedoch auch so gearbeitet werden, daß man Phenol und Dichlorbenzol und dazu Alkalihydroxid in Form einer wäßrigen Lösung vorlegt, dieses Gemisch azeotrop entwässert und dann nach Zugabe des polaren aprotischen Lösungsmittels das gesamte Reaktionsgemisch bei der gewünschten Temperatur reagieren läßt. Das polare aprotische Lösungsmittel kann in einem solchen Fall, wenn es höher siedet als das Azeotrop Wasser-Dichlorbenzol auch schon zu Beginn der Phenolatbildung zugesetzt werden. Selbstverständlich ist es weiterhin möglich, zusätzliche Schleppmittel für das azeotrop zu entfernende Wasser, wie Toluol oder Xylol, einzusetzen. Dadurch laßt sich die azeotrope Entwässerung des Reaktionsgemisches auch bei niedrigeren Temperaturen durchführen.

Die Aufarbeitung des fertigen Reaktionsgemisches kann ebenfalls in verschiedenen Varianten erfolgen. Beispielsweise ist es möglich, zunächst das polare aprotische Lösungsmittel sowie das überschüssige Dichlorbenzol und etwa vorhandenes freies Phenol abzudestillieren. Sodann wird der salzhaltige Rückstand mit Wasser behandelt, überschüssiges Phenolat durch Ansäuern in Phenol übergeführt und die nach einer Phasentrennung entstandene organische Phase destillativ aufgearbeitet.

In einer weiteren Aufarbeitungsform wird der nach dem Abdestillieren des polaren aprotischen Lösungsmittels, des Dichlorbenzols und des etwa vorhandenen freien Phenols erhaltene salzhaltige Rückstand durch Filtration, gegebenenfalls nach Zugabe eines nicht mit Wasser mischbaren Lösungsmittels, wie Benzol, Toluol oder Xylol, vom Salz befreit. Anschließend wäscht man das Filtrat mit verdünnter Natronlauge und Wasser, zieht das Lösungsmittel (beispielsweise im Vakuum) ab und destilliert das zurückbleibende Rohprodukt im Hochvakuum.

Die nach dem erfindungsgemäßen Verfahren herstellbaren chlorierten Diphenylether sind wertvolle chemische Zwischenprodukte im Pharma- und Pflanzenschutzbereich (EP 51 235, EP 65 485, EP 126 430). Weiterhin wurden Chlordiphenylether auch als Wachstumsregulatoren vorgeschlagen (US 3.371.120, US 3.472.782).

### Beispiel 1

### 3,4′-Dichlor-diphenylether

In einem 2 l-Autoklav wurden 257 g 4-Chlorphenol, 588 g m-Dichlorbenzol, 168 g 50-%ige wäßrige Kaliumhydroxidlösung und 700 g N-Methylpyrrolidon (NMP) über einen Wasserabscheider 3 Stunden azeotrop entwässert. Während dieser Entwässerung stieg die Sumpftemperatur von 132 bis 193°C. Anschließend wurde der Autoklav geschlossen und 17 Stunden bei 220°C gerührt. Es stellte sich ein Druck von ca. 2 bar ein. Nach dem Erkalten wurden NMP, überschüssiges m-Dichlorbenzol und 4-Chlorphenol abdestilliert. Zum Rückstand gab man 800 ml Wasser, rührte 15 Min. bei 70°C und säuerte mit verdünnter Schwefelsäure an. Nach dem Abtrennen wurde die organische Phase destillativ aufgearbeitet.

Man erhielt 308,2 g (86 %) der obigen Verbindung.

### Beispiel 2

### 2,4′-Dichlor-diphenylether

257 g 4-Chlorphenol, 588 g o-Dichlorbenzol, 168 g 50 %ige wäßrige Kaliumhydroxidlösung und 700 g NMP wurden wie in Beispiel 1 behandelt. Die Enddestillation lieferte 321 g (89,5 %) der obigen Verbindung.

### Beispiel 3

### 2-Chlor-4′-tert.-butyl-diphenylether

300 g 4-tert.-Butylphenol, 588 g o-Dichlorbenzol, 168 g 50-%ige wäßrige Kaliumhydroxidlösung und 800 g NMP wurden wie in Beispiel 1 behandelt. Man isolierte 358,9 g (91,8 %) der obigen Verbindung.

### Beispiel 4

### 4-Chlor-3-methyl-3′-chlor-diphenylether

285 g 4-Chlor-3-methyl-phenol, 588 g m-Dichlorbenzol, 168 g 50-%ige wäßrige Kaliumhydroxidlösung und 700 g NMP wurden wie in Beispiel 1 behandelt. Man isolierte 304,1 g (80,2 %) der obigen Verbidung.

## Patentansprüche

1. Verfahren zur Herstellung von chlorierten Diphenylethern der Formel 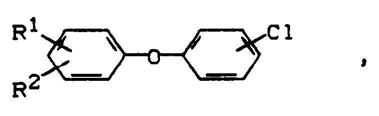 worin
R¹ für Wasserstoff, C₁-C₆-Alkyl, C₃-C₇-Cycloalkyl oder C₁-C₆-Alkoxy,
R² für Wasserstoff oder Chlor stehen,
dadurch gekennzeichnet, daß man 1 Mol eines Phenolats der Formel 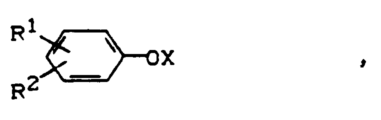 worin
X ein Alkalimetallkation ist und
R¹ und R² die obige Bedeutung haben,
mit 1,1 bis 10 Mol eines Dichlorbenzols der Formel 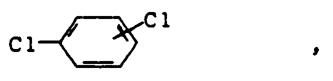 in einem polaren aprotischen Lösungsmittel im Temperaturbereich von 190 bis 240°C, bevorzugt 205 bis 225°C, in Abwesenheit von Metallkatalysatoren umsetzt.

2. Verfahren nach Anspruch 1, dadurch gekennzeichnet, daß als aprotisches Lösungsmittel eines oder mehrere aus der Gruppe von Dimethylformamid, Dimethylacetamid, Hexamethylphosphorsäuretriamid, N-Methylpyrrolidon, Dimethylsulfoxid, Sulfolan, Diethylenglykol-dimethylether, Glykol-dimethylether, Ethylencarbonat und Propylencarbonat, bevorzugt aus der Gruppe von N-Methyl-pyrrolidon, Dimethylformamid, Dimethylacetamid und Dimethylsulfoxid, eingesetzt wird.

3. Verfahren nach Anspruch 2, dadurch gekennzeichnet, daß pro Gew.-Teil Phenolat 1,5 bis 10 Gew.-Teile, bevorzugt 2 bis 6 Gew.-Teile Lösungsmittel eingesetzt werden.

4. Verfahren nach Anspruch 1, dadurch gekennzeichnet, daß 1,2 bis 5 Mol, bevorzugt 1,8 bis 3,5 Mol, Dihalogenbenzol pro Mol Phenolat eingesetzt werden.

5. Verfahren nach Anspruch 1, dadurch gekennzeichnet, daß neben dem als Phenolat gebundenen Phenol freies Phenol in einer Menge von 0,05 bis 0,6 Mol, bevorzugt 0,15 bis 0,4 Mol, pro Mol des als Phenolat gebundenen Phenols eingesetzt wird.

6. Verfahren nach Anspruch 1, dadurch gekennzeichnet, daß das Phenolat im Gemisch mit anderen Reaktionsteilnehmern aus Phenol und wäßriger Alkalimetallhydroxidlösung gebildet wird und das enthaltene Wasser azeotrop abdestilliert wird, bevor die Umsetzung begonnen wird.

## Claims

1. Process for the preparation of chlorinated diphenyl ethers of the formula 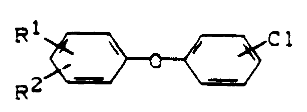 in which
R¹ represents hydrogen, C₁-C₆-alkyl, C₃-C₇-cycloalkyl or C₁-C₆-alkoxy, and
R² represents hydrogen or chlorine,
characterized in that 1 mol of a phenolate of the formula 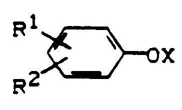 in which
X is an alkali metal cation and
R¹ and R² have the above meaning,
is reacted with 1.1 to 10 mols of a dichlorobenzene of the formula 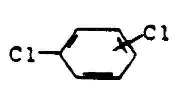 in a polar aprotic solvent in the temperature range from 190 to 240°C, preferably 205 to 225°C, in the absence of metal catalysts.

2. Process according to Claim 1, characterized in that the aprotic solvent used is one or more from the group comprising dimethylformamide, dimethylacetamide, hexamethylphosphoric triamide, N-methylpyrrolidone, dimethyl sulphoxide, sulpholane, diethylene glycol dimethyl ether, glycol dimethyl ether, ethylene carbonate and propylene carbonate, preferably from the group comprising N-methylpyrrolidone, dimethylformamide, dimethylacetamide and dimethyl sulphoxide.

3. Process according to Claim 2, characterized in that 1.5 to 10 parts by weight, preferably 2 to 6 parts by weight, of solvent are used per part by weight of phenolate.

4. Process according to Claim 1, characterized in that 1.2 to 5 mols, preferably 1.8 to 3.5 mols, of dihalogenobenzene are used per mol of phenolate.

5. Process according to Claim 1, characterized in that, in addition to the phenol bound as phenolate, free phenol is used in an amount of 0.05 to 0.6 mol, preferably 0.15 to 0.4 mol, per mol of the phenol bound as phenolate.

6. Process according to Claim 1, characterized in that the phenolate in the mixture with other reactants is formed from phenol and aqueous alkali metal hydroxide solution and the water contained is azeotropically distilled off, before the reaction is started.

## Revendications

1. Procédé de production d'éthers de diphényle chlorés de formule 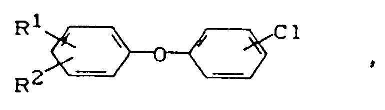 dans laquelle
R¹ représente l'hydrogène, un groupe alkyle en C₁ à C₆, cycloalkyle en C₃ à C₇ ou alkoxy en C₁ à C₆,
R² est l'hydrogène ou le chlore,
caractérisé en ce qu'on fait réagir 1 mole d'un phénolate de formule 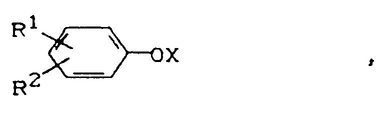 dans laquelle
X est un cation de métal alcalin et
R¹ et R² ont la définition indiquée ci-dessus,
avec 1,1 à 10 moles d'un dichlorobenzène de formule 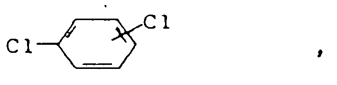 dans un solvant aprotique polaire, dans une plage de températures de 190 à 240°C, de préférence de 205 à 225°C, en l'absence de catalyseurs métalliques.

2. Procédé suivant la revendication 1, caractérisé en ce qu'on utilise comme solvant aprotique un ou plusieurs solvants du groupe du diméthylformamide, du diméthylacétamide, du triamide d'acide hexaméthylphosphorique, de la N-méthylpyrrolidone, du diméthylsulfoxyde, du sulfolane, de l'éther diméthylique du diéthylèneglycol, de l'éther diméthylique de glycol, du carbonate d'éthylène et du carbonate de propylène, de préférence du groupe de la N-méthylpyrrolidone, du diméthylformamide, du diméthylacétamide et du diméthylsulfoxyde.

3. Procédé suivant la revendication 2, caractérisé en ce qu'on utilise, par partie en poids de phénolate, 1,5 à 10 parties en poids, de préférence 2 à 6 parties en poids, de solvant.

4. Procédé suivant la revendication 1, caractérisé en ce qu'on utilise, par mole de phénolate, 1,2 à 5 moles, de préférence 1,8 à 3,5 moles, de dihalogénobenzène.

5. Procédé suivant la revendication 1, caractérisé en ce qu'on utilise, à côté du phénol lié à l'état de phénolate, du phénol libre en une quantité de 0,05 à 0,6 mole, de préférence de 0,15 à 0,4 mole, par mole du phénol lié comme phénolate.

6. Procédé suivant la revendication 1, caractérisé en ce que le phénolate est formé en mélange avec d'autres partenaires réactionnels à partir de phénols et d'une solution aqueuse d'hydroxyde de métal alcalin et l'eau présente est chassée par distillation azéotropique avant le début de la réaction.
